# EUROPEAN PATENT APPLICATION

(11) **EP 2 894 168 A1**
(43) Date of publication of application: **15.07.2015**
(21) Application number: 12763905.2
(22) Date of filing: 28.03.2012
(51) Int. Cl.: C07K 19/00, C07K 14/605, A61K 38/26, A61P 3/10

(54) **GLUCAGON-LIKE PEPTIDE-1 ANALOGUE MONOMER AND DIMER, PREPARATION METHOD THEREFOR AND APPLICATION THEREOF**

(30) Priority: 29.03.2011 CN 201110076380
(71) Applicant: Tianjin Institute Of Pharmaceutical Research, Nankai District, Tianjin 300193 (CN)
(72) Inventor: GONG, Min, Tianjin 300193 (CN); XU, Weiren, Tianjin 300193 (CN); TANG, Lida, Tianjin 300193 (CN); FU, Gang, Tianjin 300193 (CN); ZOU, Meixiang, Tianjin 300193 (CN); WU, Jiang, Tianjin 300193 (CN)
(74) Representative: Coombes, Catherine Ann
(86) International application number: PCT/CN2012/073173
(87) International publication number: WO 2012/130136

(57) **Abstract**

Provided are a glucagon-like peptide-1 (GLP-1) analogue monomer and dimmer, a preparation method thereof, and an application thereof. The GLP-1 analogue monomer comprises one cysteine; and the dimer is formed by two monomer molecules connected via an intermolecular disulfide bond formed by the cysteine. The GLP-1 monomer comprising cysteine has the following general formula: ⁷HAEX₁₀TFTSX₁₅VSSYLEX₂₂X₂₃AAKEFIX₃₀WLX₃₃KGRG³⁷, wherein X₁₀ is glycine or cysteine, X₁₅ is aspartate or cysteine, X₂₂ is glycine or cysteine, X₂₃ is glutamine or cysteine, X₃₀ is alanine or cysteine, and X₃₃ is valine or cysteine; and only one of X₁₀, X₁₅, X₂₂, X₂₃, X₃₀, and X₃₃ is cysteine. The glucagon-like peptide-1 analogue dimer of the present invention has an in vivo half-life of more than 8 to 96 hours, thus facilitating clinical promotion and application.

## Description

### TECHNICAL FIELD

The present invention relates to the field of the medicaments associated with diabetes, specifically to a glucagon-like peptide-1 (GLP-1) analogue dimer with a prolonged half-life of GLP-1 in vivo. The present invention also relates to a preparation method of the GLP-1 analogue dimer and use of the GLP-1 analogue dimer in the manufacture of a medicament for treating diabetes, and a medicament for treating and/or preventing obesity.

### BACKGROUND ART

The glucagon-like peptide-1 (hereinafter referred to as GLP-1) is a polypeptide consisting of 37 amino acids mainly secreted by small intestinal L cells, and the active forms of GLP-1 are GLP-1(7-37)OH and GLP-1(7-36)NH₂ (Mojsov S, J Clin Invest. 1987 Feb; 79(2): 616-9). GLP-1 can significantly reduce the blood glucose after meals in human, stimulate the production of insulin, and meanwhile also play a role in reducing body weight without causing hypoglycemia (Drucker D J, Diabetes. 1998 Feb; 47(2): 159-69). Recent research also shows that GLP-1 has a pancreas regeneration effect (Drucker D J, 2003 Dec; 144(12): 5145-8). Moreover, GLP-1 is a fully humanized polypeptide, and thus possesses a great advantage in safety as a clinical drug. However, GLP-1(7-37) needs to be administered by injection for many times every day due to its serum half-life of only 3-5 minutes, and thus results in much inconvenience in the clinical use.

Recently, there are many researches using the GLP-1 analogue fusion protein technology to resolve the problem regarding the residence time of GLP-1 analogue in vivo (CN90101167.3, CN200710018734.2, CN200410054397.9, CN01820232.2, CN200380110152.7, CN200510039265.3, CN200610127237.1 and CN200910009642.7). However, the existing technologies are still far away from the ideal clinical goals, and generally fail to reach the clinical standard. Liraglutide, recently produced by Novo Norisk, is a GLP-1 analogue based on the modification of GLP-1 with palmitic acid, and has come into the market in America in 2009. However, Liraglutide also has the problem of a short half-life, and its dosage form still needs to be injected daily.

Therefore, a problem of the short half-life of GLP-1 in vivo needs to be solved currently.

### DISCOSLURE OF THE INVENTION

In view of the defects that the clinically used GLP-1 analogues have short residence time in vivo and needs to be injected daily, one object of the present invention is to provide a GLP-1 analogue dimer and monomer thereof with a longer half-life.

Another object of the present invention is to provide a method for preparing the GLP-1 analogue dimer and monomer thereof.

Yet another object of the present invention is to provide use of the GLP-1 analogue dimer in the manufacture of a medicament for treating diabetes, and use of the GLP-1 analogue dimer in the manufacture of a medicament for treating and/or preventing obesity.

Still another object of the present invention is to provide a pharmaceutical composition comprising the GLP-1 analogue dimer as an active component,
wherein the pharmaceutical composition further comprises one or more pharmaceutically acceptable auxiliary materials, and the pharmaceutical composition is preferably an injection, further preferably a freeze-dried powder or a solution for injection.

The technical solutions for achieving the above objects are as follows:
In one aspect, the present invention provides a glucagon-like peptide-1 analogue monomer having the following general formula I:

   ⁷HAEX₁₀T FTSX₁₅V SSYLE X₂₂X₂₃AAK EFIX₃₀W LX₃₃KGR G³⁷;

   wherein X₁₀ is glycine or cysteine, X₁₅ is aspartic acid or cysteine, X₂₂ is glycine or cysteine, X₂₃ is leucine or cysteine, X₃₀ is alanine or cysteine, and X₃₃ is valine or cysteine; and only one of X₁₀, X₁₅, X₂₂, X₂₃, X₃₀, and X₃₃ is cysteine.

The glucagon-like peptide-1 analogue monomer as mentioned above is selected from:
SEQ ID NO 1: ⁷HAECT FTSDV SSYLE GQAAK EFIAW LVKGR G³⁷,
SEQ ID NO 2: ⁷HAEGT FTSCV SSYLE GQAAK EFIAW LVKGR G³⁷,
SEQ ID NO 3 : ⁷HAEGT FTSCV SSYLE GQAAK EFIAW LVKGR G³⁷,
SEQ ID NO 4: ⁷HAEGT FTSDV SSYLE CQAAK EFIAW LVKGR G³⁷,
SEQ ID NO 5: ⁷HAEGT FTSDV SSYLE GCAAK EFIAW LVKGR G³⁷,
SEQ ID NO 6: ⁷HAEGT FTSDV SSYLE GQAAK EFICW LVKGR G³⁷, and
SEQ ID NO 7: ⁷HAEGT FTSDV SSYLE GQAAK EFIAW LCKGR G³⁷.

In another aspect, the present invention provides a glucagon-like peptide-1 analogue dimer, which is formed by connecting two monomer molecules of the general formula I, wherein the two monomers for forming the dimer can be same or different;

Preferably, the analogue dimer is formed by connecting the monomers via a disulfide bond formed via cysteine.

In yet another aspect, the present invention provides a method for preparing the glucagon-like peptide-1 analogue monomer or dimer, which comprises solid-phase synthesis of the glucagon-like peptide-1 analogue monomer containing cysteine in accordance with Fmoc strategy.

Preferably, as for the preparation of the glucagon-like peptide-1 analogue dimer, the method further comprises a step of forming a disulfide bond between the cysteine in the resulting glucagon-like peptide-1 analogue monomers.

In another aspect, the present invention provides use of the glucagon-like peptide-1 analogue monomer or dimer as described above in the manufacture of a medicament for treating and/or preventing diabetes and diabetes related diseases.

In yet another aspect, the present invention provides use of the glucagon-like peptide-1 analogue monomer or dimer as described above in the manufacture of a medicament for treating and/or preventing obesity and obesity related diseases.

Preferably, the obesity and obesity related diseases are the obesity caused by diabetes and the obesity related diseases caused by diabetes.

In a further aspect, the present invention provides a pharmaceutical composition comprising the glucagon-like peptide-1 analogue monomer or dimer as described above.

Preferably, the pharmaceutical composition further comprises one or more pharmaceutically acceptable auxiliary materials.

Preferably, the pharmaceutical composition is an injection.

More preferably, the pharmaceutical composition is a freeze-dried powder or a solution for injection.

In another aspect, the present invention provides the glucagon-like peptide-1 analogue monomer or dimer according to the present invention for treating and/or preventing diabetes and diabetes related diseases, or obesity and obesity related diseases.

In yet another aspect, the present invention provides a method for treating and/or preventing diabetes and diabetes related diseases, or obesity and obesity related diseases, comprising administering to a subject a therapeutically effective amount of the glucagon-like peptide-1 analogue monomer according to the present invention, or the glucagon-like peptide-1 analogue dimer according to the present invention.

Preferably, the obesity and obesity related diseases are the obesity caused by diabetes and the obesity related diseases caused by diabetes.

Preferably, the subject is mammal, and the mammal is preferably human.

The following are the detailed description of the present invention:

### (1) GLP-1 analogue monomer containing cysteine

The general formula of the GLP-1 analogue monomer containing cysteine according to the present invention is as follows:

⁷HAEX₁₀T FTSX₁₅V SSYLE X₂₂X₂₃AAK EFIX₃₀ W LX₃₃KGR G³⁷.

Such as humanized GLP-1 sequence ⁷HAEGT FTSDV SSYLE GQAAK EFIAW LVKGR G³⁷.

The above GLP-1 analogue monomer containing cysteine is an artificial synthesized sequence, in which the amino acid at position 10, 15, 22, 23, 30 or 33 is modified by replacing the original amino acid with cysteine respectively. The specific sequences are as follows:
SEQ ID NO 1: ⁷HAECT FTSDV SSYLE GQAAK EFIAW LVKGR G³⁷,
SEQ ID NO 2: ⁷HAEGT FTSCV SSYLE GQAAK EFIAW LVKGR G³⁷,
SEQ ID NO 3 : ⁷HAEGT FTSCV SSYLE GQAAK EFIAW LVKGR G³⁷,
SEQ ID NO 4: ⁷HAEGT FTSDV SSYLE CQAAK EFIAW LVKGR G³⁷,
SEQ ID NO 5: ⁷HAEGT FTSDV SSYLE GCAAK EFIAW LVKGR G³⁷,
SEQ ID NO 6: ⁷HAEGT FTSDV SSYLE GQAAK EFICW LVKGR G³⁷,
SEQ ID NO 7: ⁷HAEGT FTSDV SSYLE GQAAK EFIAW LCKGR G³⁷.

The GLP-1 analogue dimer is formed by connecting the GLP-1 analogue monomers containing cysteine by a disulfide bond formed via cysteine. One of the above amino acid sequences can be selected as one monomer of the analogue dimer, while the other monomer of the analogue dimer can be the same sequence or other sequences.

### (2) The pharmaceutical composition of the present invention

The pharmaceutical composition of the present invention may be prepared from the GLP-1 analogue dimer together with one or more pharmaceutically acceptable auxiliary materials including water soluble filling agent, pH regulator, stabilizing agent, water for injection, osmotic pressure regulator and so on.

The auxiliary materials for water soluble filling agent of the present invention are one or more selected from the group consisting of mannitol, low molecular weight dextran, sorbitol, polyethylene glycol, glucose, lactose, galactose and so on.

The pH regulator is one or more selected from the group consisting of non-volatile acids, such as citric acid, phosphoric acid, lactic acid, tartaric acid, hydrochloric acid and etc.; physiologically acceptable organic or inorganic acids, bases and/or salts and so on, such as potassium hydroxide, sodium hydroxide or potassium hydroxide or ammonium hydroxide, sodium carbonate or potassium carbonate or ammonium carbonate, sodium bicarbonate, potassium bicarbonate or ammonium bicarbonate.

The stabilizing agent is one or more selected from the group consisting of EDTA-2Na, sodium thiosulfate, sodium metabisulfite, sodium sulfite, dipotassium hydrogen phosphate, sodium bicarbonate, sodium carbonate, arginine, glutamic acid, polyethylene glycol 6000, polyethylene glycol 4000, sodium dodecyl sulfate or trihydroxymethyl aminomethane and so on. Preferably, the stabilizing agent is selected from one or more of the group consisting sodium metabisulfite, dipotassium hydrogen phosphate, arginine, polyethylene glycol 6000 and trihydroxymethyl aminomethane.

The osmotic pressure regulator is sodium chloride and/or potassium chloride.

### (3) Preparation method of injections

The pharmaceutical composition of the present invention can be administered by intramuscular, intravenous, or subcutaneous injection, and the preferable dosage form is a freeze-dried powder or a solution for injection.

### Preparation method of freeze-drying injection:

To an appropriate amount of the GLP-1 analogue dimer solution, water soluble filling agent, stabilizing agent, osmotic pressure regulator and the like are added, and an appropriate amount of water for injection is added. The pH value is adjusted to 4-8 so as to dissolve the materials therein. The resulting solution is diluted to a proper concentration by adding water. 0.1-0.5% of active carbon is added to the solution and removed after the solution is stirred at 0 - 10 °C for 10-20 minutes The solution is filtered with microfiltration membrane to remove bacteria. The filtrate is subpackaged, and then fabricated in accordance with freeze-drying method as a white, loose and blocky substance, which is sealed to obtain the freeze-drying injection. Each specification contains the GLP-1 analogue dimer between 5µg and 1mg.

### Preparation method of injection solution:

To an appropriate amount of GLP-1 analogue dimer solution, water soluble filling agent, stabilizing agent, osmotic pressure regulator and the like are added, and an appropriate amount of water for injection is added. The pH value is adjusted to 4-8 so as to dissolve the materials therein. The resulting solution is diluted to a proper concentration by adding water. 0.1-0.5% of active carbon is added to the solution, and then removed after the solution is stirred at 0 - 10 °C for 10-20 minutes. The solution is filtered with microfiltration membrane to remove bacteria. The filtrate is subpackaged, and sealed to obtain the injection solution. Each specification contains the GLP-1 analogue dimer between 5µg and 1mg.

### (3) Use of pharmaceutical composition

The GLP-1 analogue dimer of the present invention can be used in the manufacture of a medicament for treating diabetes. Specifically, the composition of the present invention can be administered in the form of intravenous, intramuscular, or subcutaneous injection. The dosage is varied depending on the subject to be treated, mode of administration, symptoms and other factors. The GLP-1 analogue dimer of the present invention is effective in a wide range of dosages. In the treatment of the adults, the dosage range is between 5µg/person and 1mg/person, administered once daily or every several days. The actual dosage should be determined by a physician according to the related conditions including the physical state of the patient to be treated, administration route, age, weight, individual response to the drug, severity of the patients' symptoms and the like. Therefore, the above dosage range does not limit the scope of the present invention in any way.

The GLP-1 analogue dimers of the present invention have overcome the problem of the short half-life of GLP-1. The half-life of the GLP-1 analogue dimers provided can reach above 8-96 hours in vivo, which is significantly longer than that of GLP-1 administrated alone (with the half-life of only 3-5 minutes), thereby are greatly favorable for the clinical spreading and application of the GLP-1 analogue dimer of the present invention.

### BRIEF DESCRIPTION OF THE DRAWINGS

The examples of the present invention are illustrated in detail below with reference to the figures, in which:
Fig. 1 shows a blood glucose reducing test of the GLP-1 analogue dimer in Example 2, wherein the time periods from left to right are SEQ1/1, SEQ1/2, SEQ1/3, SEQ1/4, SEQ1/5, SEQ1/6, SEQ1/7 and saline successively;
Fig. 2 shows a blood glucose reducing test of the GLP-1 analogue dimer in Example 3, wherein the time periods from left to right are SEQ2/1, SEQ2/2, SEQ2/3, SEQ2/4, SEQ2/5, SEQ2/6, SEQ2/7 and saline successively;
Fig. 3 shows a blood glucose reducing test of the GLP-1 analogue dimer in Example 4, wherein the time periods from left to right are SEQ3/4, SEQ3/5, SEQ3/6, SEQ3/7 and saline successively;
Fig. 4 shows a blood glucose reducing test of the GLP-1 analogue dimer in Example 5, wherein the time periods from left to right are SEQ4/5, SEQ4/6, SEQ4/7 and saline successively;
Fig. 5 shows a blood glucose reducing test of the GLP-1 analogue dimer in Example 6, wherein the time periods from left to right are SEQ5/6, SEQ5/7 and saline successively;
Fig. 6 shows a blood glucose reducing test of the GLP-1 analogue dimer in Example 7, wherein the time periods from left to right are SEQ6/6, SEQ6/7 and saline successively;
Fig. 7 shows a blood glucose reducing test of the GLP-1 analogue dimer in Example 8, wherein the time periods from left to right are SEQ7/7 and saline successively.

### BEST MODE FOR CARRYING OUT THE INVENTION

It may be understood that the specific embodiments described herein are illustrated by way of example and not as the limitation of the present invention. The main technical features of the present invention may be used to various embodiments without departing from the scope of the present invention. A person skilled in the art will realize or can confirm that many equivalents can be used into the specific steps described herein merely by conventional experiments. These equivalents are deemed as in the scope of the present invention and are covered by the appended claims.

In the following examples, various processes and methods which are not described in detail are conventional methods well known in the art.

The mice used in the following Examples are 6 to 8 week Kunming mice, each of them weighing about 20g.

### Example 1: solid-phase synthesis of polypeptide

Using the method of solid phase polypeptide synthesis in accordance with Fmoc strategy, the synthesis of GLP-1 analogue monomer containing cysteine of the present invention is performed using the CS 336X type apparatus produced by CSBio Company. The method of synthesis is performed in accordance with the manufacturer's equipment specifications.

The obtained GLP-1 analogue monomer containing cysteine is purified on a HPLC C18 semi-preparative column with acetonitrile as the mobile phase. Dry powder of the GLP-1 analogue monomer containing cysteine is obtained through desalination and lyophilization. The disulfide bond in this Example is formed by ammonium bicarbonate or other reducing agent.

### Example 2: related blood glucose reducing function of GLP-1 analogue dimer (formed from the monomer of SEQ ID NO 1 and other GLP-1 analogue monomer)

The GLP-1 analogue dimers used in this Example are as follows:
The GLP-1 analogue dimer formed from the GLP-1 analogue monomers containing cysteine of SEQ ID NO 1 and SEQ ID NO 1, respectively (SEQ1/1);
The GLP-1 analogue dimer formed from the GLP-1 analogue monomers containing cysteine of SEQ ID NO 1 and SEQ ID NO 2, respectively (SEQ1/2);
The GLP-1 analogue dimer formed from the GLP-1 analogue monomers containing cysteine of SEQ ID NO 1 and SEQ ID NO 3, respectively (SEQ1/3);
The GLP-1 analogue dimer formed from the GLP-1 analogue monomers containing cysteine of SEQ ID NO 1 and SEQ ID NO 4, respectively (SEQ1/4);
The GLP-1 analogue dimer formed from the GLP-1 analogue monomers containing cysteine of SEQ ID NO 1 and SEQ ID NO 5, respectively (SEQ1/5);
The GLP-1 analogue dimer formed from the GLP-1 analogue monomers containing cysteine of SEQ ID NO 1 and SEQ ID NO 6 respectively (SEQ1/6);
The GLP-1 analogue dimer formed from the GLP-1 analogue monomers containing cysteine of SEQ ID NO 1 and SEQ ID NO 7 respectively (SEQ1/7).

The above analogue dimers (for a total of seven) were dissolved into saline respectively, to a concentration of 1 mg/mL for each, and were subcutaneously injected into the mice (200 µL per mouse, 6 mice per group, and the mice are purchased from Shanghai Laboratory Animal Center of Chinese Academy of Sciences). In this Example, the blank control group is subcutaneously injected with saline. After 30 minutes from the injection, 400 µg glucose was injected into each mouse. The blood glucose levels of the mice were measured at 2 hours, 24 hours, 48 hours, 72 hours and 96 hours after the injection of the glucose respectively. After the first measurement of the blood glucose, the same dosage of glucose was administered again at two hours before each measurement of the blood glucose.

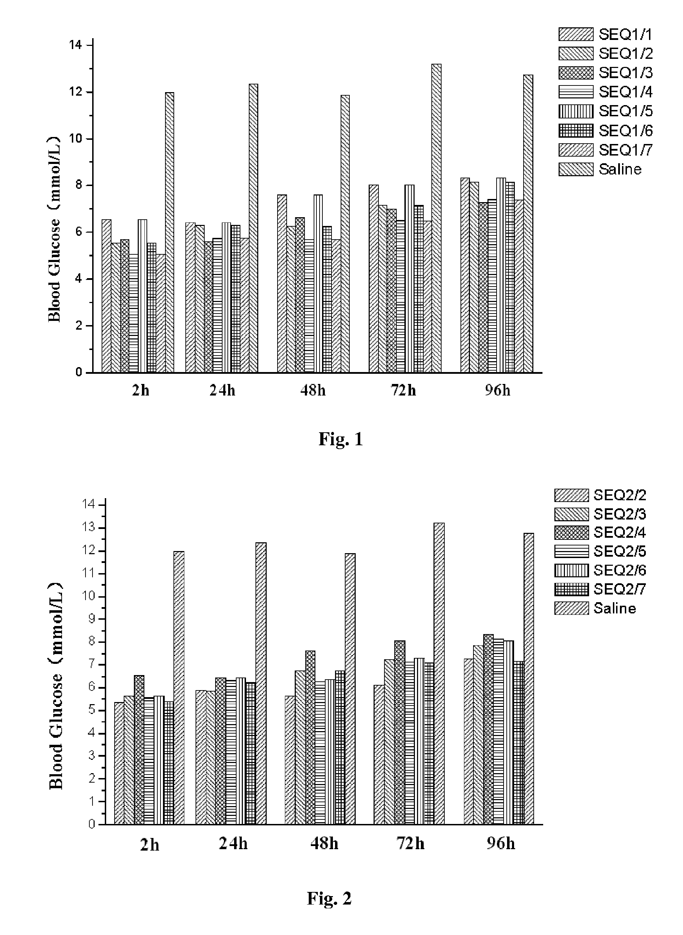

The results are shown in Figure 1, and indicate that the above GLP-1 analogue dimers have longer half-lives in vivo.

### Example 3: related blood glucose reducing function of GLP-1 analogue dimer (formed from the monomer of SEQ ID NO 2 and other GLP-1 analogue monomer)

The GLP-1 analogue dimers used in this Example are as follows:
The GLP-1 analogue dimer formed from the GLP-1 analogue monomers containing cysteine of SEQ ID NO 2 and SEQ ID NO 2 respectively (SEQ2/2);
The GLP-1 analogue dimer formed from the GLP-1 analogue monomers containing cysteine of SEQ ID NO 2 and SEQ ID NO 3 respectively (SEQ2/3);
The GLP-1 analogue dimer formed from the GLP-1 analogue monomers containing cysteine of SEQ ID NO 2 and SEQ ID NO 4 respectively (SEQ2/4);
The GLP-1 analogue dimer formed from the GLP-1 analogue monomers containing cysteine of SEQ ID NO 2 and SEQ ID NO 5 respectively (SEQ2/5);
The GLP-1 analogue dimer formed from the GLP-1 analogue monomers containing cysteine of SEQ ID NO 2 and SEQ ID NO 6 respectively (SEQ2/6);
The GLP-1 analogue dimer formed from the GLP-1 analogue monomers containing cysteine of SEQ ID NO 2 and SEQ ID NO 7 respectively (SEQ2/7).

The above analogue dimers (for a total of six) were dissolved into saline respectively, to a concentration of 1 mg/mL for each, and were subcutaneously injected into mice (200 µL per mouse, 6 mice per group, and the mice are purchased from Shanghai Laboratory Animal Center of Chinese Academy of Sciences). In this Example, the blank is subcutaneously injected with saline. After 30 minutes from the injection, 400 µg glucose was injected into each mouse, and the blood glucose levels of the mice were measured at 2 hours, 24 hours, 48 hours, 72 hours and 96 hours after the injection of the glucose respectively. After the first measurement of the blood glucose, the same dosage of glucose was administered again at two hours before each measurement of the blood glucose.

The results are shown in Figure 2, and indicate that the above GLP-1 analogue dimers have long half-lives in vivo.

### Example 4: related blood glucose reducing function of GLP-1 analogue dimer (formed from the monomer of SEQ ID NO 3 and other GLP-1 analogue monomer)

The GLP-1 analogue dimers used in this Example are as follows:
The GLP-1 analogue dimer formed from the GLP-1 analogue monomers containing cysteine of SEQ ID NO 3 and SEQ ID NO 4 respectively (SEQ3/4);
The GLP-1 analogue dimer formed from the GLP-1 analogue monomers containing cysteine of SEQ ID NO 3 and SEQ ID NO 5 respectively (SEQ3/5);
The GLP-1 analogue dimer formed from the GLP-1 analogue monomers containing cysteine of SEQ ID NO 3 and SEQ ID NO 6 respectively (SEQ3/6);
The GLP-1 analogue dimer formed from the GLP-1 analogue monomers containing cysteine of SEQ ID NO 3 and SEQ ID NO 7 respectively (SEQ3/7).

The above analogue dimers (for a total of four) were dissolved into saline respectively, to a concentration of 1 mg/mL for each, and were subcutaneously injected into mice (200 µL per mouse, 6 mice per group, and the mice are purchased from Shanghai Laboratory Animal Center of Chinese Academy of Sciences). In this Example, the blank is subcutaneously injected with saline. After 30 minutes from the injection, 400 µg of glucose was injected into each mouse, and the blood glucose levels of the mice were measured at 2 hours, 24 hours, 48 hours, 72 hours and 96 hours after the injection of the glucose respectively. After the first measurement of the blood glucose, the same dosage of glucose was administered again at two hours before each measurement of the blood glucose.

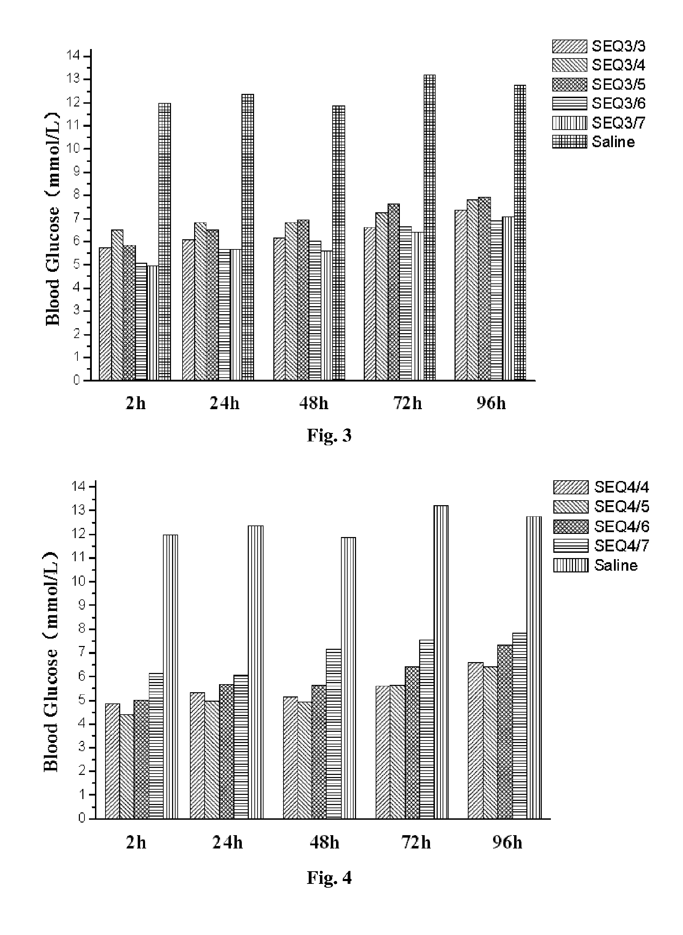

The results are shown in Figure 3, and indicate that the above GLP-1 analogue dimers have long half-lives in vivo.

### Example 5: related blood glucose reducing function of GLP-1 analogue dimer (formed from the monomer of SEQ ID NO 4 and other GLP-1 analogue monomer)

The GLP-1 analogue dimers used in this Example are as follows:
The GLP-1 analogue dimer formed from the GLP-1 analogue monomers containing cysteine of SEQ ID NO 4 and SEQ ID NO 5 respectively (SEQ4/5);
The GLP-1 analogue dimer formed from the GLP-1 analogue monomers containing cysteine of SEQ ID NO 4 and SEQ ID NO 6 respectively (SEQ4/6);
The GLP-1 analogue dimer formed from the GLP-1 analogue monomers containing cysteine of SEQ ID NO 4 and SEQ ID NO 7 respectively (SEQ4/7).

The above analogue dimers (for a total of three) were dissolved into saline respectively, to a concentration of 1 mg/mL for each, and were subcutaneously injected into mice (200 µL per mouse, 6 mice per group, and the mice are purchased from Shanghai Laboratory Animal Center of Chinese Academy of Sciences). In this Example, the blank is subcutaneously injected with saline. After 30 minutes from the injection, 400 µg glucose was injected into each mouse, and the blood glucose levels of the mice were measured at 2 hours, 24 hours, 48 hours, 72 hours and 96 hours after the injection of the glucose respectively. After the first measurement of the blood glucose, the same dosage of glucose was administered again at two hours before each measurement of the blood glucose.

The results are shown in Figure 4, and indicate that the above GLP-1 analogue dimers have long half-lives in vivo.

### Example 6: related blood glucose reducing function of GLP-1 analogue dimer (formed from the monomer of SEQ ID NO 5 and other GLP-1 analogue monomer)

The GLP-1 analogue dimers used in this Example are as follows:
The GLP-1 analogue dimer formed from the GLP-1 analogue monomers containing cysteine of SEQ ID NO 5 and SEQ ID NO 6 respectively (SEQ5/6);

The GLP-1 analogue dimer formed from the GLP-1 analogue monomers containing cysteine of SEQ ID NO 5 and SEQ ID NO 7 respectively (SEQ5/7).

The above analogue dimers (for a total of two) were dissolved into saline respectively, to a concentration of 1 mg/mL for each, and were subcutaneously injected into mice (200 µL per mouse, 6 mice per group, and the mice are purchased from Shanghai Laboratory Animal Center of Chinese Academy of Sciences). In this Example, the blank is subcutaneously injected with saline. After 30 minutes from the injection, 400 µg glucose was injected into each mouse, and the blood glucose levels of the mice were measured at 2 hours, 24 hours, 48 hours, 72 hours and 96 hours after the injection of the glucose respectively. After the first measurement of the blood glucose, the same dosage of glucose was administered again at two hours before each measurement of the blood glucose.

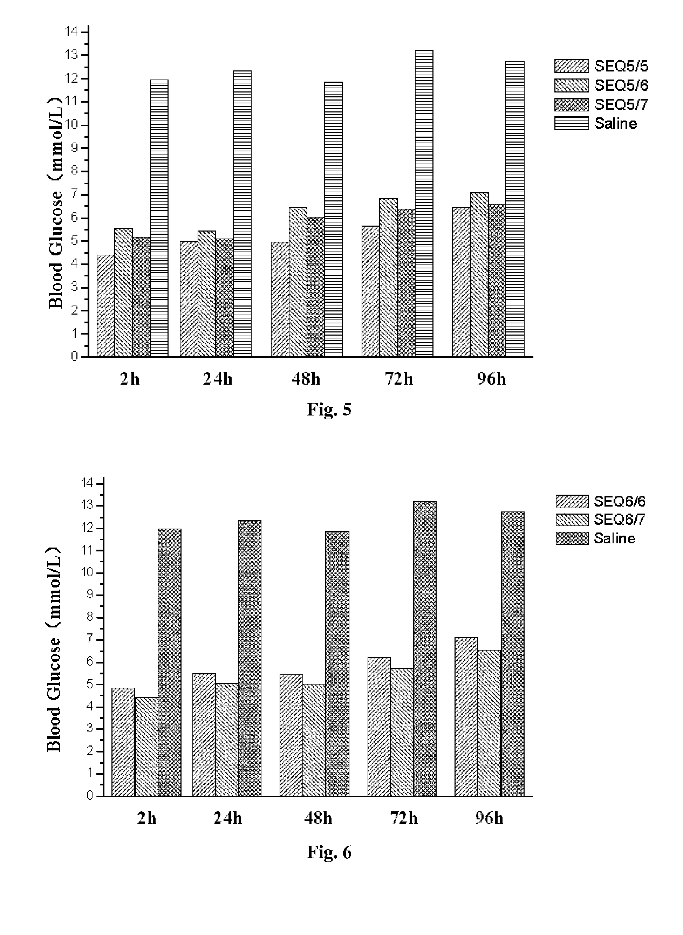

The results are shown in Figure 5, and indicate that the above GLP-1 analogue dimers have long half-lives in vivo.

### Example 7: related blood glucose reducing function of GLP-1 analogue dimer (formed from the monomer of SEQ ID NO 6 and other GLP-1 analogue monomer)

The GLP-1 analogue dimer used in this Example is as follows:
The GLP-1 analogue dimer formed from the GLP-1 analogue monomers containing cysteine of SEQ ID NO 6 and SEQ ID NO 7 respectively (SEQ6/7).

The above analogue dimer was dissolved into saline to a concentration of 1 mg/mL, and was subcutaneously injected into mice (200 µL per mouse, 6 mice per group, and the mice are purchased from Shanghai Laboratory Animal Center of Chinese Academy of Sciences). In this Example, the blank is subcutaneously injected with saline. After 30 minutes from the injection, 400 µg glucose was injected into each mouse, and the blood glucose levels of the mice were measured at 2 hours, 24 hours, 48 hours, 72 hours and 96 hours after the injection of the glucose respectively. After the first measurement of the blood glucose, the same dosage of glucose was administered again at two hours before each measurement of the blood glucose.

The results are shown in Figure 6, and indicate that the above GLP-1 analogue dimers have long half-lives in vivo.

### Example 8: related blood glucose reducing function of GLP-1 analogue dimer (formed from the monomers of SEQ ID NO 7 and SEQ ID NO 7 analogue)

The GLP-1 analogue dimer used in this Example is as follows:
The GLP-1 analogue dimer formed from the GLP-1 analogue monomers containing cysteine of SEQ ID NO 7 and SEQ ID NO 7 respectively (SEQ7/8).

The above analogue dimer was dissolved into saline to a concentration of 1 mg/mL, and was subcutaneously injected into mice (200 µL per mouse, 6 mice per group, and the mice are purchased from Shanghai Laboratory Animal Center of Chinese Academy of Sciences). In this Example, the blank is subcutaneously injected with saline. After 30 minutes from the injection, 400 µg glucose was injected into each mouse, and the blood glucose levels of the mice were measured at 2 hours, 24 hours, 48 hours, 72 hours and 96 hours after injection of the glucose respectively. After the first measurement of the blood glucose, the same dosage of glucose was administered again at two hours before each measurement of the blood glucose.

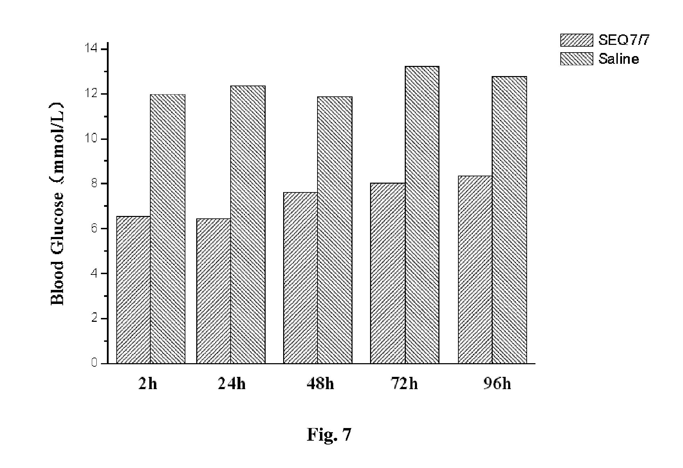

The results are shown in Figure 7, and indicate that the above GLP-1 analogue dimers have long half-lives in vivo.

## Claims

1. A glucagon-like peptide-1 analogue monomer, **characterized in that** the monomer has the following general formula:
⁷HAEX₁₀T FTSX₁₅V SSYLE X₂₂X₂₃AAK EFIX₃₀W LX₃₃KGR G³⁷;
wherein X₁₀ is glycine or cysteine, X₁₅ is aspartic acid or cysteine, X₂₂ is glycine or cysteine, X₂₃ is leucine or cysteine, X₃₀ is alanine or cysteine, and X₃₃ is valine or cysteine; and only one of X₁₀, X₁₅, X₂₂, X₂₃, X₃₀, and X₃₃ is cysteine.

2. The glucagon-like peptide-1 analogue monomer according to claim 1, **characterized in that** the monomer is selected from:
SEQ ID NO 1: ⁷HAECT FTSDV SSYLE GQAAK EFIAW LVKGR G³⁷,
SEQ ID NO 2: ⁷HAEGT FTSCV SSYLE GQAAK EFIAW LVKGR G³⁷,
SEQ ID NO 3 : ⁷HAEGT FTSCV SSYLE GQAAK EFIAW LVKGR G³⁷,
SEQ ID NO 4: ⁷HAEGT FTSDV SSYLE CQAAK EFIAW LVKGR G³⁷,
SEQ ID NO 5: ⁷HAEGT FTSDV SSYLE GCAAK EFIAW LVKGR G³⁷,
SEQ ID NO 6: ⁷HAEGT FTSDV SSYLE GQAAK EFICW LVKGR G³⁷, and
SEQ ID NO 7: ⁷HAEGT FTSDV SSYLE GQAAK EFIAW LCKGR G³⁷.

3. A glucagon-like peptide-1 analogue dimer, **characterized in that** the dimer is formed by connecting two monomers according to claim 1 or 2, and the monomers for forming the dimer can be the same or different;
preferably, the dimer is formed by the monomers connected via disulfide bonds formed by cysteines.

4. A method for preparing the glucagon-like peptide-1 analogue monomer according to claim 1 or 2 or the glucagon-like peptide-1 analogue dimer according to claim 3, **characterized in that** the method comprises solid-phase synthesis of the glucagon-like peptide-1 analogue monomer containing cysteines in accordance with Fmoc strategy,
preferably, as for the preparation of the glucagon-like peptide-1 analogue dimer, the method further comprises a step of forming disulfide bonds between the obtained glucagon-like peptide-1 analogue monomers via cysteines.

5. Use of the glucagon-like peptide-1 analogue monomer according to claim 1 or 2, or the glucagon-like peptide-1 analogue dimer according to claim 3 in the manufacture of a medicament for treating and/or preventing diabetes and diabetes related diseases.

6. Use of the glucagon-like peptide-1 analogue monomer according to claim 1 or 2, or the glucagon-like peptide-1 analogue dimer according to claim 3 in the manufacture of a medicament for treating and/or preventing of obesity and obesity related diseases,
preferably, the obesity and obesity related diseases are obesity caused by diabetes and obesity related diseases caused by diabetes.

7. A pharmaceutical composition, **characterized in that** the pharmaceutical composition comprises the glucagon-like peptide-1 analogue monomer according to claim 1 or 2, or the glucagon-like peptide-1 analogue dimer according to claim 3.

8. The pharmaceutical composition according to claim 7, **characterized in that** the pharmaceutical composition further comprises one or more pharmaceutically acceptable carrier;
preferably, the pharmaceutically acceptable carrier is selected from water soluble filling agent, pH regulator, stabilizing agent, water for injection and osmotic pressure regulator;
more preferably, the water soluble filling agent is one or more selected from the group consisting of mannitol, low molecular weight dextran, sorbitol, polyethylene glycol, glucose, lactose and galactose;
more preferably, the pH regulator is physiologically acceptable acids, bases and/or salts, which is preferably one or more selected from the group consisting of:
non-volatile acids, such as citric acid, phosphoric acid, lactic acid, tartaric acid or hydrochloric acid,
bases, such as potassium hydroxide, sodium hydroxide or potassium hydroxide or ammonium hydroxide,
salts, such as sodium carbonate or potassium carbonate or ammonium carbonate, sodium bicarbonate, potassium bicarbonate or ammonium bicarbonate;
more preferably, the stabilizing agent is one or more selected from of the group consisting of EDTA-2Na, sodium thiosulfate, sodium metabisulfite, sodium sulfite, dipotassium hydrogen phosphate, sodium bicarbonate, sodium carbonate, arginine, glutamic acid, polyethylene glycol 6000, polyethylene glycol 4000, sodium dodecyl sulfate or trihydroxymethyl aminomethane and so on; further preferably, the stabilizing agent is one or more selected from of group consisting of sodium metabisulfite, dipotassium hydrogen phosphate, arginine, polyethylene glycol 6000 and trihydroxymethyl aminomethane;
more preferably, the osmotic pressure regulator is selected from sodium chloride and/or potassium chloride.

9. The pharmaceutical composition according to claim 7 or 8, **characterized in that** the pharmaceutical composition is an injection;
preferably, the pharmaceutical composition is a freeze-dried powder or a solution injection.

10. A method for treating and/or preventing diabetes and diabetes related diseases, or obesity and obesity related diseases, comprising administering to a subject a therapeutically effective amount of the glucagon-like peptide-1 analogue monomer according to claim 1 or 2, or the glucagon-like peptide-1 analogue dimer according to claim 3,
preferably, the obesity and obesity related diseases are obesity caused by diabetes and obesity related diseases caused by diabetes; and
more preferably, the subject is mammal, and the mammal is preferably human.


SEQUENCE LISTING

<110>  Tianjin Institue of Pharmaceutical Research

<120>  Glucagon-Like Peptide-1 Analogue Monomer and Dimer, Preparation
Method thereof and Use thereof

<130>  EIC12310001P-EP

<160>  7

<170>  PatentIn version 3.3

<210>  1
<211>  31
<212>  PRT
<213>  Artificial Sequence

<220>
<223>  SEQ ID NO 1

<400>  1

His Ala Glu Cys Thr Phe Thr Ser Asp Val Ser Ser Tyr Leu Glu Gly
1               5                   10                  15

Gln Ala Ala Lys Glu Phe Ile Ala Trp Leu Val Lys Gly Arg Gly
            20                  25                  30

<210>  2
<211>  31
<212>  PRT
<213>  Artificial Sequence

<220>
<223>  SEQ ID NO 2

<400>  2

His Ala Glu Gly Thr Phe Thr Ser Cys Val Ser Ser Tyr Leu Glu Gly
1               5                   10                  15

Gln Ala Ala Lys Glu Phe Ile Ala Trp Leu Val Lys Gly Arg Gly
            20                  25                  30

<210>  3
<211>  31
<212>  PRT
<213>  Artificial Sequence

<220>
<223>  SEQ ID NO 3

<400>  3

His Ala Glu Gly Thr Phe Thr Ser Cys Val Ser Ser Tyr Leu Glu Gly
1               5                   10                  15

Gln Ala Ala Lys Glu Phe Ile Ala Trp Leu Val Lys Gly Arg Gly
            20                  25                  30

<210>  4
<211>  31
<212>  PRT
<213>  Artificial Sequence

<220>
<223>  SEQ ID NO 4

<400>  4

His Ala Glu Gly Thr Phe Thr Ser Asp Val Ser Ser Tyr Leu Glu Cys
1               5                   10                  15

Gln Ala Ala Lys Glu Phe Ile Ala Trp Leu Val Lys Gly Arg Gly
            20                  25                  30

<210>  5
<211>  31
<212>  PRT
<213>  Artificial Sequence

<220>
<223>  SEQ ID NO 5

<400>  5

His Ala Glu Gly Thr Phe Thr Ser Asp Val Ser Ser Tyr Leu Glu Gly
1               5                   10                  15

Cys Ala Ala Lys Glu Phe Ile Ala Trp Leu Val Lys Gly Arg Gly
            20                  25                  30

<210>  6
<211>  31
<212>  PRT
<213>  Artificial Sequence

<220>
<223>  SEQ ID NO 6

<400>  6

His Ala Glu Gly Thr Phe Thr Ser Asp Val Ser Ser Tyr Leu Glu Gly
1               5                   10                  15

Gln Ala Ala Lys Glu Phe Ile Cys Trp Leu Val Lys Gly Arg Gly
            20                  25                  30

<210>  7
<211>  31
<212>  PRT
<213>  Artificial Sequence

<220>
<223>  SEQ ID NO 7

<400>  7

His Ala Glu Gly Thr Phe Thr Ser Asp Val Ser Ser Tyr Leu Glu Gly
1               5                   10                  15

Gln Ala Ala Lys Glu Phe Ile Ala Trp Leu Cys Lys Gly Arg Gly
            20                  25                  30